# EUROPEAN PATENT APPLICATION

(11) **EP 4 545 063 A1**
(43) Date of publication of application: **30.04.2025**
(21) Application number: 24190615.5
(22) Date of filing: 24.07.2024
(51) Int. Cl.: A61K 8/34, A61K 8/36, A61K 8/46, A61K 8/49, A61P 17/14, A61Q 7/00

(54) **COSMETICAL USE OF A TOPICALLY APPLICABLE RINSE-OF COMPOSITION (RC)**

(30) Priority: 23.10.2023 EP 23205280
(71) Applicant: Wella Germany GmbH, 64295 Darmstadt (DE)
(72) Inventor: SPRINGOB, Christian, 64295 Darmstadt (DE); RIEMANN, Ingo, 64295 Darmstadt (DE)
(74) Representative: Baier, Martin

(57) **Abstract**

The present invention relates to the cosmetic use of a topically applicable rinse-off composition (RC) comprising as components (A) sandal pentanol (3-methyl-5-(2,2,3-trimethylcyclopent-3-en-1-yl)pentan-2-ol)and /or one or more sandal pentanol analog(s), (B) caffeine, (C) at least one C10-C18 fatty acid and/or salts thereof, (D) at least one surfactant, and (E) at least one cosmetically acceptable solvent for (i) improving hair structure, and (ii) hair loss reduction. In another aspect the present invention relates to a non-therapeutic method for (i) improving hair structure, and (ii) hair loss using the topically applicable rinse-off composition (RC).

## Description

The present invention relates to the cosmetic use of a topically applicable rinse-off composition (RC) comprising as components (A) sandal pentanol (3-methyl-5-(2,2,3-trimethylcyclopent-3-en-1-yl)pentan-2-ol)and /or one or more sandal pentanol analog(s), (B) caffeine, (C) at least one C10-C18 fatty acid and/or salts thereof, (D) at least one surfactant, and (E) at least one cosmetically acceptable solvent for (i) improving hair structure, and (ii) hair loss reduction. In another aspect the present invention relates to a non-therapeutic method for (i) improving hair structure, and (ii) hair loss using the topically applicable rinse-off composition (RC).

### BACKGROUND OF THE INVENTION

The growth of human hair occurs in a known manner according to a natural growth rhythm including a growth phase (anagen), a transition phase (catagen), a resting phase (telogen) and a release phase (exogen). A normal natural hair cycle begins when a hair bottom end is ejected or cast away in exogen phase or prior to beginning of a new anagen phase. During the anagen phase the cells in the root of the hair are dividing rapidly so that new hair is formed, with a typical growth rate for hair on the scalp of approximately 0.3 to 0.4 millimeters per day. Anagen phase lasts about 2-6 years. Following the anagen phase, the catagen phase denotes a short transition phase wherein hair growth is stopped and the cells that produce hair growth are cut off from blood supply. Catagen phase lasts approximately ten days. During telogen phase, which lasts about 100 days, the individual hairs are still retained in the follicles but are no longer actively growing. Finally, at exogen phase the individual hairs are ejected or released. Exogen phase lasts approximately 2-5 months.

During normal hair growth, 80 to 100 hairs fall out from the scalp per day. This number is an approximate value, which can occur over a shorter time and also can be greater or smaller, without causing fear regarding natural hair growth. However, when the amount of hair falling out daily exceeds this approximate normal amount, an irreversible or reversible hair loss is occurring. WO2022/112419 discloses a composition comprising sandal pentanol, caffeine, and one of more C10-C18 fatty acids. This composition can be used for reducing hair loss.

Any loss of hair volume over the time is generally perceived as "thinning of hair", but such a volume loss may be caused by different phenomena. One obvious reason for perceived thinner hair is hair loss, i.e. the number or areal density of hair follicles producing hair decreases. Another reason for perceived thinner hair is alterations in the growth cycle. For example, the percentage of hairs in anagen phase and the duration of the anagen phase may diminish, resulting primarily in shorter hairs but perceived as an overall loss of hair volume.

Another reason for perceived thinner hair is an alteration of the diameter or cross-section of the hair, i.e. a thinning of the individual hairs. Structure and size of human head hair or scalp hair vary depending on ethnic origin. Asian hair has a round, even shape and an average diameter from 80 to 120 micrometers. Caucasian hair is oval in shape, with 60 to 80 micrometers considered a typical average diameter for hair. African hair has a flattened oval shape, with average diameters from 50 to 70 micrometers considered typical. Average diameters also vary depending on age, with increasing diameters until the early 40s, and after that age the average diameter decreasing with an increasing rate.

Therefore, an object of the present invention is the provision of care products suitable for hair loss reduction in combination with improving hair structure, in particular comprising increasing the diameter or cross-section of hair or improving keratin expression. Further, there is a need for care products suitable for improving the structure of hair affected by age-related hair thinning. In particular, there is a need for such products which can be administered topically.

This object is achieved by the cosmetic use of a topically applicable rinse-off composition (RC) comprising as components
(A) sandal pentanol ((3-methyl-5-(2,2,3-trimethylcyclopent-3-en-1-yl)pentan-2-ol) and /or one or more sandal pentanol analog(s),
(B) caffeine,
(C) at least one C10-C18 fatty acid and/or salts thereof,
(D) at least one surfactant, and
(E) at least one cosmetically acceptable solvent
   for
   (i) improving hair structure, and
   (ii) hair loss reduction,

wherein the topically applicable rinse-off composition (RC) comprises

component (A) in an amount of from 0.010 to 10 % by weight,

component (B) in an amount of from 0.010 to 10 % by weight,

component (C) in an amount of from 0.010 to 5 % by weight,

component (D) in an amount of from 5.0 to 30 % by weight, and component (E) in an amount of from 45 to 94.97 % by weight,

wherein the % by weight values are based on the total weight of the topically applicable rinse-off composition (RC).

According to a preferred aspect, subject matter of the present invention is the cosmetic use of the topically applicable rinse-off composition (RC) for improving hair structure (i) in combination with hair loss reduction (ii), in particular the structure of mammalian hair such as human hair.

According to the present invention, it has now surprisingly been found that the combination of sandal pentanol, caffeine, and at least one C10-C18 fatty acid and/or salts thereof is effective for improving hair structure (i) and hair loss reduction (ii). The present inventors found out that the combination of sandal pentanol, caffeine, and at least one C10-C18 fatty acid and/or salts thereof is suitable for hair loss reduction (ii) in combination with improving hair structure (i), wherein improving hair structure preferably comprises increasing the cross-section of the hair.

Further, the present inventors found out that the combination of sandal pentanol caffeine, and at least one C10-C18 fatty acid and/or salts thereof is suitable for improving hair structure, wherein improving hair structure (i) preferably comprises increasing the expression of keratin, in particular the expression of keratin 85 in combination with hair loss reduction (ii). Further, the present inventors found out that the combination of sandal pentanol caffeine, and at least one C10-C18 fatty acid and/or salts thereof is suitable for improving the structure of hair affected by age-related hair thinning.

The cosmetic use of the topically applicable rinse-off composition (RC) of the present invention is suitable for improving hair structure (i) in combination with hair loss reduction (ii). In an aspect, the cosmetic use of the topically applicable rinse-off composition (RC) of the present invention is suitable for improving hair structure, wherein improving hair structure (i) comprises increasing the cross-section of the hair in combination with hair loss reduction (ii). In another aspect, the cosmetic use of the topically applicable rinse-off composition (RC) of the present invention is suitable for improving hair structure (i), wherein improving hair structure comprises increasing the expression of keratin, for example the expression of keratin 85 in combination with hair loss reduction (ii). In yet another aspect, the cosmetic use of the topically applicable rinse-off composition (RC) of the present invention are suitable for improving the structure of hair affected by age-related hair thinning in combination with hair loss reduction.

The cosmetic use of the topically applicable rinse-off composition (RC) of the present invention further is suitable for topical administration on the human scalp. The cosmetic use of the topically applicable rinse-off composition (RC) of the present invention are suitable both for cosmetic and therapeutic use in improving hair structure (i) in combination with hair loss reduction (ii).

### DEFINITIONS

In the present specification, the terms "sandal pentanol" and "Sandalore" are used interchangeably. Sandal pentanol is a synthetic odorant having a fragrance similar to sandalwood, and which consequently is used in perfumes, emollients and skin cleaning agents as a less expensive ingredient mimicking sandalwood scent. Sandal pentanol, as well as structurally similar analogs thereof such as the compound named brahmanol, are alcohols having a chemical structure quite distinct from the ingredients of natural sandalwood oil, which are extracted by steam distillation from matured sandalwood trees, and are well-known valued components for perfumes. The IUPAC denomination of sandal pentanol is 3-methyl-5-(2,2,3-trimethylcyclopent-3-en-1-yl)pentan-2-ol.

As used in the specification and the appended claims, the singular forms "a," "an" and "the" include plural referents unless the context clearly dictates otherwise. The term "may" in the context of this application means "is permitted to" or "is able to" and is a synonym for the term "can." The term "may" as used herein does not mean possibility or chance.

The term "and/or" in the context of this application means one or the other or both. For example, an aqueous solution of A and/or B means an aqueous solution of A alone, an aqueous solution of B alone and an aqueous solution of a combination of A and B.

The term "about" is understood to mean ±10 percent of the recited number, numbers or range of numbers. According to embodiments, the term "about" is understood to mean ±2 percent of the recited number, numbers or range of numbers.

As used herein, the term "optionally" means that the corresponding step or feature may or may not be present. It includes both possibilities.

The terms "parts-by-weight" (PBW) and "parts-by-volume" (PBV) are used in their usual meaning. When used in a mixed context, for example an amount of a component indicated in PBW in admixture with a component indicated in PBV, the terms PBW and PBV as used herein denote the amount in grams of the component indicated in PBW relative to a volume in milliliters of the amount of the component indicated in PBV.

The term "composition" as used herein denotes a mixture or blend comprising at least two components. Typical compositions comprise at least one functional component (such as, for example, an active ingredient such as sandal pentanol) and at least one solvent component (such as water or an alcohol). A composition may be present in any suitable physical form, including for example liquids of varying viscosities, gels, foams, creams, and the like. The term "composition" may denote a ready-to-use product unless the context dictates otherwise.

"Ready-to-use scalp-and-hair treatment product" denotes the form in which the composition is actually applied to human scalp, or a skin region comprising facial hair.

The term "essentially free" of a particular component denotes an amount of less than 1.0% by weight of the respective component in an entity such as a composition, based on the total weight of the entity. In particular, the term "essentially free" denotes an amount of less than 0.5% by weight, for example less than 0.1% by weight, such as less than 0.01 % by weight of the respective component in the entity.

The term "free" of a particular component denotes an amount below the detection limit, assuming that detectability is on a ppm (parts per million) basis (by weight). In particular, the term "free" of a particular component denotes an amount below 50 ppm by weight, for example less than 10 ppm by weight, such as less than 5 ppm by weight of the respective component in the entity.

The term "consisting essentially of" denotes the absence of additional, not specifically recited components in an entity such as a composition in an amount of more than 1.0% by weight, based on the total weight of the entity. In particular, the term "consisting essentially of" denotes the absence of additional, not specifically recited components in an amount of more than 0.5% by weight, for example more than 0.1% by weight, such as more than 0.01% by weight in an entity. It goes without saying that any additional, not specifically recited component is compatible with the specifically recited components and does not interfere with the function thereof.

The term "consisting of" denotes the absence of additional, not specifically recited components in an entity such as a composition in an amount below the detection limit, assuming that detectability is on a ppm (parts per million) basis (by weight). In particular, the term "consisting of" denotes the absence of additional, not specifically recited components in an amount of more than 50 ppm by weight, for example more than 10 ppm by weight, such as more than 10 ppm by weight in an entity. It goes without saying that any additional, not specifically recited component is compatible with the specifically recited components and does not interfere with the function thereof.

The term "hair" as used herein denotes generally mammalian hair, but in particular human hair. More specifically, for the purposes of the present invention the term "hair" denotes human "head hair" or "scalp hair", and human "facial hair". The term "facial hair" encompasses beard hair, eyebrows and eyelashes.

The term "improved hair structure" denotes an improvement in one or more structural phenomena of hair. For example, "improved hair structure" encompasses an improved/increased "diameter" or "cross-section" of the hair "as grown". In another example, "improved hair structure" encompasses improved/increased keratin expression, in particular improved/increased expression of KRT85. The term "keratin 85" or "KRT85" denotes human keratin 85.

The term "age-related hair-thinning" denotes a perceived loss of the volume of human hair caused by a decrease of the diameter or cross-section of the hair, which decrease occurs in an age-dependent fashion with an onset age typically in the early 40's. Scientific studies have demonstrated that the age-dependent decrease of the diameter or cross-section of hair correlates with a decreased expression of keratins, including a decreased expression of KRT85.

The term "KRT85 Expression" denotes the expression of human keratin 85. Just beyond the proliferative zone with stem cells (many studies refer to as the 'germative matrix' or Zone A) KRT85 occurs as one of the first keratins expressed in the cortical cells forming short filaments (paired with KRT35 or KRT31) in the cytoplasm in the 'precortical hair matrix'. KRT85 promotes lateral association of short intermediate filaments (IFs) into bundles. These initial bundles are the first signs of keratin ultrastructure and are normally found in the cortex about level with the top of the dermal papilla. These intermediate filaments are always found as tightly aligned bundles. As cortex cells move into Zone B final fibre diameter and shape is determined as the Henle's layer hardens (Inner Root Sheath). In Zone C (start of the keratogenous region) the final shape and position is maintained. This suggests that KRT85 expression correlates directly with hair shaft quality, with an increased KRT85 expression resulting in an increased cross-section of the hair.

### DETAILED DESCRIPTION OF THE INVENTION

The inventors of the present invention surprisingly found that a combination of sandal pentanol (component (A)), caffeine (component (B)), and at least one C10-C18 fatty acid and/or salts thereof (component (C)) is effective for improving hair structure (i) and hair loss reduction (ii). The inventors of the present invention surprisingly found that sandal pentanol (component (A)), caffein (component (B)), and at least one C10-C18 fatty acid and/or salts thereof (component (C)) are effective in a synergistic manner for improving hair structure (i) and hair loss reduction (ii).

### Component (A)

In the present case the terms "(A)", "component (A)", "sandal pentanol and/or one or more sandal pentanol analog(s)" are used synonymously, and therefore have preferably the same meaning.

Sandal pentanol is one of the active ingredients used according to the present disclosure for improving hair structure (i) and hair loss reduction (ii). Sandal pentanol is a synthetic odorant used as an ingredient mimicking sandalwood scent. Sandal pentanol is an alcohol having a chemical structure quite distinct from the ingredients of natural sandalwood oil which usually are extracted by steam distillation of wood from matured sandalwood trees.

The IUPAC denomination of sandal pentanol is 3-methyl-5-(2,2,3-trimethylcyclopent-3-en-1-yl)pentan-2-ol. Sandal pentanol has been shown to have a stimulatory effect on the olfactory receptor OR2AT4, which is expressed in the epithelium of human hair follicles.

Analogs or derivatives, respectively, of sandal pentanol are believed to have a similar effect on improving hair structure (i) and hair loss reduction and may be used in the present disclosure alternatively or additionally to sandal pentanol. Sandal pentanol analogs suitable as active ingredients include:
- (4Z)-3-methyl-5-(2,2,3-trimethylcyclopent-3-en-1-yl)pent-4-en-2-ol (sandal pentenol)
- 1-methyl-2-((1,2.2-trimethylbicyclo(3.1.0)hex-3-yl)methyl)-cyclopropane-methanol
   (sandal cyclopropane)
- (E)-3,3-dimethyl-5-(2,2,3-trimethylcyclopent-3-en-1-yl)pent-4-en-2-ol (santol pentenol)
- 2-methyl-4-(2,2,3-trimethyl-3-cyclopenten-1-yl)butanol (sandal cyclopentane)
- (E)-2-ethyl-4-(2,2,3-trimethylcyclopent-3-en-1 -yl)but-2-en-1 -ol (sandalrome)
- (E)-2-methyl-4-(2,2,3-trimethyl-3-cyclopenten-1-yl)-2-buten-1-ol (sandal butenol)

According to embodiments, the topically applicable rinse-off composition (RC) used according to the present disclosure may comprise one or more of the above sandal pentanol analog(s) in addition to the active ingredient sandal pentanol. If present, sandal pentanol plus the one or more sandal pentanol analogs are present in total amounts as indicated below for sandal pentanol alone.

According to other embodiments, the topically applicable rinse-off composition (RC) used according to the present disclosure may comprise sandal pentanol as an active ingredient, and being essentially free of, or free of the above sandal pentanol analogs.

According to further, less preferred embodiments, the topically applicable rinse-off composition (RC) used according to the present disclosure may comprise one or more of the above sandal pentanol analogs substituting for the active ingredient sandal pentanol.

The amount of sandal pentanol in the topically applicable rinse-off composition (RC) used according to the present disclosure is not particularly critical. Typically, the sandal pentanol will be present in an amount of from 0.010 to 10.0 percent-by-weight, based on the weight of the topically applicable rinse-off composition (RC). If the amount is below 0.010 percent-by-weight, the effect sought by the present disclosure may be difficult to achieve. Amounts above 10.0 percent-by-weight may not further enhance the effect but contribute to higher costs of the material resources for the topically applicable rinse-off composition (RC) used according to the present disclosure. According to embodiments, the sandal pentanol may be present in an amount of 0.050 to 5.0 percent-by-weight, based on the weight of the topically applicable rinse-off composition (RC). According to more specific embodiments, the sandal pentanol may be present in an amount of 0.10 to 2.0 percent-by-weight, based on the weight of the topically applicable rinse-off composition (RC). According to even more specific embodiments, the sandal pentanol may be present in an amount of 0.50 to 1.50 percent-by-weight, based on the weight of the topically applicable rinse-off composition (RC).

If analogs of sandal pentanol are present, either replacing at least in part the sandal pentanol, or in addition to sandal pentanol, the total amount of sandal pentanol plus one or more of sandal pentanol analogs (as indicated above) is within the ranges indicated above for sandal pentanol exclusively.

### Component (B)

In the present case the terms "(B)", "component (B)", and "caffeine" are used synonymously, and therefore have preferably the same meaning.

Caffeine is another active ingredient used according to the present disclosure for improving hair structure (i), and hair loss reduction (ii). Caffeine is a plant alkaloid also known under the names theine, methyltheobromine and guaranine. The IUPAC denomination is 1,3,7-trimethylxanthine. Caffeine is a well-known stimulant which is present for example in coffee, black tea, but also in cocoa solids and products containing same (in particular dark chocolate), or cola nuts. Caffeine has a vasodilating effect, thereby increasing blood and nutrients supply to the hair roots. Studies have shown that caffeine enhances the hair shaft elongation (making the hair root bigger), prolonges anagen duration (increasing the hair growth phase) and stimulates the proliferation of hair matrix keratinocytes (increasing keratin production).

The amount of caffeine in the topically applicable rinse-off composition (RC) used according to the present disclosure is not particularly critical. If present, the caffeine typically will be present in an amount of from 0.010 to 10.0 percent-by-weight, based on the weight of the topically applicable rinse-off composition (RC). If the amount is below 0.010 percent-by-weight, the effect sought by the present disclosure may be difficult to achieve. Amounts above 10.0 percent-by-weight may not further enhance the effect but contribute to higher costs of the material resources for the topically applicable rinse-off composition (RC) used according to the present disclosure.

According to embodiments, the caffeine may be present in an amount of 0.050 to 5.0 percent-by-weight, based on the weight of the topically applicable rinse-off composition (RC). According to more specific embodiments, the caffeine may be present in an amount of 0.10 to 2.0 percent-by-weight, based on the weight of the topically applicable rinse-off composition (RC). According to even more specific embodiments, the caffeine may be present in an amount of 0.50 to 1.5 percent-by-weight, based on the weight of the topically applicable rinse-off composition (RC).

### Component (C)

In the present case the terms "(C)", "component (C)", "at least one C10-C18 fatty acid and/or salts thereof" are used synonymously, and therefore have preferably the same meaning. "At least one C10-C18 fatty acid and/or salts thereof means exactly one C10-C18 fatty acid and/or salts thereof and also mixtures of two or more different C10-C18 fatty acids and/or salts thereof.

Another active ingredient used according to the present disclosure for improving hair structure (i), and hair loss reduction (ii) are C10-C18 fatty acids. The fatty acid(s) used for improving hair structure (i), and hair loss reduction (ii) is/are one or more linear and/or branched, saturated and/or unsaturated C10-C18 fatty acids. Typically, the one or more C10-C18 fatty acids will be linear fatty acids. According to embodiments, the C10-C18 fatty acid(s) may be selected from capric acid (C10:0), lauric acid (C12:0), myristic acid (C14:0), isomyristic acid (C14:0), palmitic acid (C16:0), palmitoleic acid (C16:1), stearic acid (C18:0), oleic acid (C18:1), linoleic acid (C18:2), linolenic acid (C18:3), parinaric acid (C18:4), petroselinic acid (C18:1), or combinations thereof, and/or salts thereof.

According to embodiments, the C10-C18 fatty acid(s) may comprise lauric acid (C12:0), myristic acid (C14:0), or a combination thereof. According to specific embodiments, the C10-C18 fatty acid(s) may be selected from lauric acid (C12:0), myristic acid (C14:0), or a combination thereof. For example, the fatty acid component of the active ingredient combination may be lauric acid (C12:0), and/or salts thereof.

According to embodiments, the fatty acid component may comprise one or more unsaturated fatty acids. According to embodiments, the C10-C18 fatty acid(s) may comprise palmitoleic acid (C16:1), oleic acid (C18:1), linoleic acid (C18:2), linolenic acid (C18:3), parinaric acid (C18:4), petroselinic acid (C18:1), or a combination thereof. For example, the fatty acid component may comprise linoleic acid (C18:2), and/or salts thereof.

According to specific embodiments, the fatty acid component may be selected from palmitoleic acid (C16:1), oleic acid (C18:1), linoleic acid (C18:2), linolenic acid (C18:3), parinaric acid (C18:4), petroselinic acid (C18:1), or a combination thereof. For example, the fatty acid component may be selected from palmitoleic acid (C16:1) and/or linoleic acid (C18:2), and/or salts thereof.

If the fatty acid component comprises one or more unsaturated fatty acids, the respective compositions preferably comprise an antioxidant in order to reduce autoxidation of the double bonds.

According to embodiments, the topically applicable rinse-off composition (RC) used according to the present disclosure may be essentially free, or free of unsaturated C10-C18 fatty acids. According to specific embodiments, compositions according to the present disclosure may be essentially free, or free of unsaturated fatty acids per se.

If present, the C10-C18 fatty acid(s) may be present in the form of free acid, or in the form of a cosmetically acceptable salt, for example in the form of an alkali metal salt, alkaline earth metal salt, ammonium salt or combination thereof. For example, if present the C10-C18 fatty acid(s) may be present in the form of the free acid(s).

The amount of C10-C18 fatty acid(s) in the topically applicable rinse-off composition (RC) used according to the present disclosure is not particularly critical. If present, the C10-C18 fatty acid(s) typically will be present in an amount of from 0.010 to 10.0 percent-by-weight, based on the weight of the the topically applicable rinse-off composition (RC). If the amount is below 0.010 percent-by-weight, the effect sought by the present disclosure may be difficult to achieve. Amounts above 10.0 percent-by-weight may not further enhance the effect but contribute to higher costs of the material resources for the topically applicable rinse-off composition (RC) used according to the present disclosure.

High amounts of C10-C18 fatty acid(s) further may require higher amounts of surfactants (component (D)) and may result in a greasy appearance or hair feel. According to embodiments, the C10-C18 fatty acid(s) may be present in an amount of 0.050 to 3.0 percent-by-weight, based on the weight of the topically applicable rinse-off composition (RC). According to more specific embodiments, the C10-C18 fatty acids may be present in an amount of 0.05 to 1 percent-by-weight, based on the weight of the topically applicable rinse-off composition (RC). According to even more specific embodiments, the C10-C18 fatty acids may be present in an amount of 0.1 to 0.3 percent-by-weight, based on the weight of the topically applicable rinse-off composition (RC) used.

### Component (D)

In the present case the terms "(D)", "component (D)" and "at least one surfactant" are used synonymously, and therefore have preferably the same meaning. The term "at least one surfactant" means exactly one surfactant and, also a mixture of two or more different surfactants.

The surfactants suitable as component (D) include anionic, cationic, amphoteric and non-ionic surfactants.

The anionic surfactants may be selected from the group consisting of salts (such as alkaline salts, for example, sodium salts, ammonium salts, amine salts, amino alcohol salts and magnesium salts) of the following compounds: alkyl sulphates, alkyl ether sulphates, alkylamido ether sulphates, alkylarylpolyether sulphates, monoglyceride sulphates; alkyl sulphonates, alkyl phosphates, alkylamide sulphonates, alkylaryl sulphonates, alpha-olefin sulphonates, paraffin sulphonates; alkyl sulphosuccinates, alkyl ether sulphosuccinates, alkylamide sulphosuccinates; alkyl sulphosuccinamates; alkyl sulphoacetates; alkyl ether phosphates; acyl sarcosinates; acyl isethionates; N-acyltaurates; and mixtures thereof. The alkyl or acyl radical of all of these various compounds, for example, comprises from 8 to 24 carbon atoms, and the aryl radical, for example, is chosen from phenyl and benzyl groups. Weakly anionic surfactants can also be used, such as alkyl-D-galactosiduronic acids and their salts, as well as polyoxyalkylenated (C6-C24) alkyl ether carboxylic acids, polyoxyalkylenated (C6-C24) alkylaryl ether carboxylic acids, polyoxyalkylenated (C6-C24) alkylamido ether carboxylic acids and their salts, for example, those comprising from 2 to 50 ethylene oxide groups, and mixtures thereof. Anionic derivatives of polysaccharides, for example carboxyalkyl ether of alkyl polyglucosides, can be also used.

Suitable anionic surfactant(s) (Component (D)) may comprise at least one anionic functional groups at their head selected from sulfate, sulfonate, and phosphate and carboxylates. In order to avoid any overlap with an active ingredient combination of the present disclosure, anionic surfactant(s) for use in the compositions according to the present disclosure exclude fatty acids or salts thereof.

Suitable alkyl sulfates include ammonium lauryl sulfate, sodium lauryl sulfate (sodium dodecyl sulfate, SLS, or SDS), and alkyl-ether sulfates, such as sodium laureth sulfate (sodium lauryl ether sulfate or SLES), and sodium myreth sulfate. Further suitable anionic surfactants may include docusate (dioctyl sodium sulfosuccinate), alkyl-aryl ether phosphate, alkyl ether phosphate, sodium lauroyl sarcosinate, ammonium laureth sulfate, disodium lauryl sulfosuccinate, and sodium lauryl sulphoacetate.

Preferred anionic surfactants may be selected from the group consisting of sodium laurylethersulfate, sodium laurethethersulfate, sodium dodecyl sulfate, ammonium laurethethersulfat, ammonium dodecyl sulfate, alkylbenzenesulfonate, and combinations thereof.

The surfactant may be a non-ionic surfactant. The non-ionic surfactant may be selected from the group consisting of lanolin alcohol, and polyoxyethylene ethers of fatty alcohols, and mixtures thereof. The non-ionic surfactant may be preferably ceteareth-n, wherein n is from 2 to 100, or from 10 to 30. Suitable nonionic surfactants are compounds that are well known (see, for example, in this respect "Handbook of Surfactants" by M. R. Porter, published by Blackie & Son (Glasgow and London), 1991, pp. 116-178). Preferred surfactants encompass non-ionic surfactants, in particular polyoxyethylated fatty alcohols or oils. Particularly preferred non-ionic surfactants include polyoxyethylated Castor Oil and hydrogenated polyoxylated Castor Oil. Specific examples of non-ionic surfactants that may be used in the compositions of the present disclosure are PEG-35 Castor Oil, PEG-40 Hydrogenated Castor Oil, and combinations thereof.

Component (D) is preferably present in an amount of from 5 to 30 % by weight, more preferably in an amount of from 10 to 25 % by weight, and particularly preferred in an amount of from 12 to 20 % by weight, wherein the % by weight values are based on the total weight of the topically applicable rinse-off composition (RC).

### Component (E)

In the present case the terms "(E)", "component (E)" and "at least one cosmetically acceptable solvent" are used synonymously, and therefore have preferably the same meaning. The term "at least one cosmetically acceptable solvent" means exactly one cosmetically acceptable solvent and, also a mixture of two or more different cosmetically acceptable solvents.

The cosmetically acceptable solvent used in the topically applicable rinse-off composition (RC) preferably is water-based, alcohol-based or water-alcohol-based. Typically, the cosmetically acceptable solvent comprises water and one or more monovalent and/or polyvalent C1-C4 alcohols. According to embodiments, the solvent comprises water and an alcohol selected from ethanol, n-propanol, isopropanol, glycerol, ethylene glycol, propylene glycol or a combination thereof.

Preferred as component (E) are water, glycerol or a mixture of water and glycerol.

Component (E) is preferably present in an amount of from 45 to 94.97 % by weight, based on the total weight of the topically applicable rinse-off composition (RC).

In a preferred embodiment component (E) comprises at least 50 % by weight, more preferred at least 70 % by weight, and particularly preferred at least 80 % by weight of water, based on the total weight of component (E) comprised in the topically applicable rinse-off composition (RC).

In another preferred embodiment component (E) comprises at least 50 % by weight, more preferred at least 70 % by weight, and particularly preferred at least 80 % by weight of glycerol, based on the total weight of component (E) comprised in the topically applicable rinse-off composition (RC).

### Optional added further components

The topically applicable rinse-off composition (RC) used according to the present disclosure may optionally comprise additional components which enhance, supplement or add to the benefit obtained by sandal pentanol alone or an active ingredient combination. Such additional optional components will be referred to in the following as supportive component, oil component and mineral component. The supportive component covers a broad spectrum of substances having diversified influences on physiologic or metabolic processes. The oil component covers natural essential oils and modified oils. Modified oils include in particular hydrogenated oils and oil constituents. The mineral component covers minerals in water-soluble or alcohol-soluble form.

According to embodiments, compositions according to the present disclosure further may comprise a supportive component. Examples of such supportive components include in particular vasodilatators, blood circulation stimulators, antioxidants, vitamins, provitamins. If present, the amount of the supportive component or combinations thereof will be in the range of from 0.0010 to 5.0, in particular from 0.010 to 3.0 percent-by-weight, based on the weight of the topically applicable rinse-off composition (RC).

The supportive component may comprise, for example, one or more of tocopherols, tocotrienols, tocopherol esters, nicotinic acid, nicotinic amides, nicotinic esters, vasodilatory plant extracts, camphor, royal jelly, biotin, vitamin B3, vitamin B12, vitamin C, vitamin D2, vitamin D3, vitamin E, vitamin K1, vitamin K2, panthenol (provitamin B5), omega-3 fatty acids, omega-6 fatty acids, folates, cannabidiol, amino acids, or combinations thereof.

According to embodiments, the supportive component includes a vasodilator selected from tocopherols (alpha-, beta-, gamma- or delta-tocopherol), in particular alpha-tocopherol, tocopherol esters (including, for example, tocopherol acetate, tocopheryl succinate, tocopherylnicotinate, tocopherylpoly(oxyethylene)-succinate), nicotinic acid, nicotinic amides, nicotinic esters, camphor; an antioxidant such as vitamin C or polyphenol(s); an inhibitor of testosterone-5-alpha-reductase such as the omega-6 fatty acid arachidonic acid; a substance providing a benefit to hair health such as nicotinic amide (niacinamide); or a combination thereof. According to embodiments, the supportive component includes tocopherol or tocopheryl acetate (vitamin E acetate), and/or niacinamide. Suitable vasodilatory plant extracts include burned or distilled nettle extract (Urtica Diocia), roast chestnut extract (Aesculus Hippocastanum), Arnica extract (Arnica Montana), or hemp extract.

The oil component may comprise, for example, peppermint oil, rosemary essential oil, lavender essential oil, tea tree oil, ylang-ylang, thyme oil, cedarwood oil, clary sage essential oil, bergamot oil, jojoba oil, sunflower oil, hydrogenated ethylhexyl olivate, or a combination thereof. If present, the oil component will be present in the compositions of the present disclosure in an amount of from 0.0010 to 1.0, in particular from 0.010 to 0.50 percent-by-weight, based on the weight of the topically applicable rinse-off composition (RC).

According to embodiments, the oil component includes peppermint oil and/or hydrogenated ethylhexyl olivate.

The mineral component includes metal ions having a beneficial effect on hair and/or scalp health. The mineral component may comprise, for example, iron, zinc, or a combination thereof. If present, the mineral component will be present in the compositions of the present disclosure in 0.0050-0.50 molar concentration, for example in 0.010-0.25 molar concentration.

The topically applicable rinse-off composition (RC) used according to the present disclosure may comprise a supportive component, an oil component or a mineral component alone or in any combination.

The topically applicable rinse-off composition (RC) used according to the present disclosure optionally may comprise cosmetically acceptable additives, which may contribute to achieve or enhance the properties or effects sought. According to embodiments, the cosmetically acceptable additives may be selected from perfumes, opacifiers, preservatives, pH modifiers, chelators, hair-care materials or skin-care materials, physiologically compatible silicone derivative compounds, light protecting agents, anti-flaking agents, hair luster-imparting agents, combability improving agents, defatting agents, moisturizers, conditioning agents, viscosity modifiers, cooling agents. The cosmetically acceptable additives may be used in any combinations, as desired or required.

Suitable cosmetically acceptable additives not specifically described below are listed in the International Cosmetics Ingredient Dictionary and Handbook, (8th ed.; The Cosmetics, Toiletry, and Fragrance Association). Particularly, vol. 2, sections 3 (Chemical Classes) and 4 (Functions), which are useful in identifying specific additives to achieve a particular purpose or multipurpose. A few of these additives are discussed below, whose disclosure is of course non-exhaustive.

Suitable amount ranges for perfumes, opacifiers, preservatives, pH modifiers, chelators, hair-care materials or skin-care materials, physiologically compatible silicone derivative compounds, light protecting agents, anti-flaking agents, hair luster-imparting agents, combability improving agents, defatting agents, moisturizers, conditioning agents, viscosity modifiers, cooling agents are indicated for additives discussed below. These cosmetically acceptable additives may be present in a total amount of up to 20.0, in particular up to 15.0 percent-by-weight, based on the weight of the topically applicable rinse-off composition (RC). Typically, the total amount of these cosmetically acceptable additives may be within a range of from 0.010 to 10.0, conveniently within a range of from 0.010 to 7.0 percent-by-weight, based on the weight of the topically applicable rinse-off composition (RC). For example, the total amount of these cosmetically acceptable additives may be within a range of from 0.010 to 5.0, conveniently within a range of from 0.010 to 3.0 percent-by-weight, based on the weight of the the topically applicable rinse-off composition (RC) used.

The topically applicable rinse-off composition (RC) used according to the present disclosure may comprise a perfume or fragrance. For example, the The topically applicable rinse-off composition (RC) may comprise from 0.0010 to 1.50, in particular 0.010-0.50 percent-by-weight perfume, based on the weight of the topically applicable rinse-off composition (RC). Perfume can provide an enhanced user experience by making the composition smell pleasant and/or invoke emotions, such as relaxing or exciting smells.

Alternatively, the topically applicable rinse-off composition (RC) used according to the present disclosure may be substantially free of perfume and/or fragrance. Some consumers prefer perfume-free compositions. The perfume may be an animal fragrance or a plant fragrance. The animal fragrance may be selected from the group consisting of musk oil, civet, castoreum, ambergris, and mixtures thereof.

The plant fragrance may be selected from the group consisting of nutmeg extract, cardomon extract, ginger extract, cinnamon extract, patchouli oil, geranium oil, orange oil, mandarin oil, orange flower extract, cedarwood, vetyver, lavandin, ylang extract, tuberose extract, sandalwood oil, bergamot oil, rosemary oil, spearmint oil, peppermint oil, lemon oil, lavender oil, citronella oil, chamomille oil, clove oil, sage oil, neroli oil, labdanum oil, eucalyptus oil, verbena oil, mimosa extract, narcissus extract, carrot seed extract, jasmine extract, olibanum extract, rose extract, and mixtures thereof.

The perfume may comprise one or more scents selected from the group consisting of acetophenone, adoxal, aldehyde C-12, aldehyde C-14, aldehyde C-18, allyl caprylate, ambroxan, amyl acetate, dimethylindane derivatives, α-amylcinnamic aldehyde, anethole, anisaldehyde, benzaldehyde, benzyl acetate, benzyl alcohol and ester derivatives, benzyl propionate, benzyl salicylate, borneol, butyl acetate, camphor, carbitol, cinnamaldehyde, cinnamyl acetate, cinnamyl alcohol, cis-3-hexanol and ester derivatives, cis-3-hexenyl methyl carbonate, citral, citronnellol and ester derivatives, cumin aldehyde, cyclamen aldehyde, cyclo galbanate, damascones, decalactone, decanol, estragole, dihydromyrcenol, dimethyl benzyl carbinol, 6,8-dimethyl-2-nonanol, dimethyl benzyl carbinyl butyrate, ethyl acetate, ethyl isobutyrate, ethyl butyrate, ethyl propionate, ethyl caprylate, ethyl cinnamate, ethyl hexanoate, ethyl valerate, ethyl vanillin, eugenol, exaltolide, fenchone, fruity esters such as ethyl 2-methyl butyrate, galaxolide, geraniol and ester derivatives, helional, 2-heptonone, hexenol, α-hexylcinnamic aldehyde, hydroxycitrolnellal, indole, isoamyl acetate, isoeugenol acetate, ionones, isoeugenol, isoamyl iso-valerate, iso E super, limonene, linalool, lilial, linalyl acetate, lyral, majantol, mayol, melonal, menthol, p-methylacetophenone, methyl anthranilate, methyl cedrylone, methyl dihydrojasmonate, methyl eugenol, methyl ionone, methyl-α-naphthyl ketone, methylphenylcarbinyl acetate, mugetanol, γ-nonalactone, octanal, phenyl ethyl acetate, phenyl-acetaldehyde dimethyl acetate, phenoxyethyl isobutyrate, phenyl ethyl alcohol, pinenes, sandalore, santalol, stemone, thymol, terpenes, triplal, triethyl citrate, 3,3,5-trimethylcyclohexanol, γ-undecalactone, undecenal, vanillin, veloutone, verdox, and mixtures thereof.

The topically applicable rinse-off composition (RC) used in accordance with the present disclosure may comprise an opacifier, which sometimes is denoted as well by the term "turbidity-inducing agent". For example, the compositions may comprise from 0.10 to 5.0, in particular 0.50-2.0 percent-by-weight opacifier, based on the weight of the the topically applicable rinse-off composition (RC). Suitable opacifiers include mineral pigments such as, for example, titanium dioxide, alumina, calcium carbonate. Other suitable opacifiers include fatty alcohols and fatty acid esters such as, for example, cetearyl alcohol, cetyl alcohol, ethylene glycol distearate, glyceryl stearate, glyceryl oleate, stearyl stearate.

The topically applicable rinse-off composition (RC) used in accordance with the present disclosure may comprise a preservative or mixture of preservatives. The compositions may comprise the preservative(s) in an amount of up to 1.0, for example from 0.0010 to 1.0, in particular from 0.010 to 0.50 percent-by-weight, based on the weight of the topically applicable rinse-off composition (RC). Cosmetically acceptable preservatives include organic acids such as para-hydroxybenzoic acid; organic acid salts such as sodium benzoate; compounds such as benzyl alcohol, phenoxyethanol, 1,3-bis(hydroxymethyl)-5,5-dimethylimidazolidine-2,4-dione; and mixtures thereof.

The topically applicable rinse-off composition (RC) used in accordance with the present disclosure may comprise a pH modifier and/or buffering agent, in a sufficient amount to effectively adjust the pH of the composition. For example, the compositions may comprise up to 1.0, for example from 0.000010 to 1.0, in particular 0.0010-0.50 percent-by-weight pH modifiers, based on the weight of the topically applicable rinse-off composition (RC). Suitable pH modifiers and/or buffering agents for use herein include, but are not limited to: ammonia, alkanolamines such as monoethanolamine, diethanolamine, triethanolamine, monopropanolamine, dipropanolamine, tripropanolamine, tripropanolamine, 2-amino-2-methyl-1-propanol, and 2-amino-2-hydroxymethyl-1,3,-propandiol and guanidium salts, alkali metal and ammonium hydroxides and carbonates, such as sodium hydroxide, sodium silicate, sodium meta silicate and ammonium carbonate, and acids such as organic and inorganic acids, for example ascorbic acid, citric acid, acetic acid or tartaric acid, phosphoric acid, hydrochloric acid, and mixtures thereof.

Preferred alkaline pH modifiers include ammonia, ethanolamines, and alkali metal hydroxides, in particular sodium hydroxide. Preferred acidic pH modifiers include hydrochloric acid, ascorbic acid, or citric acid.

The topically applicable rinse-off composition (RC) used according to the present disclosure may further comprise one or more chelators (also known as "chelating agent", "sequestering agent", "sequestrant", or chelants) in an amount sufficient to reduce the amount of metals available to interact with formulation components. Chelators are well known in the art and a non-exhaustive list thereof can be found in AE Martell & RM Smith, Critical Stability Constants, Vol. 1, Plenum Press, New York & London (1974) and AE Martell & RD Hancock, Metal Complexes in Aqueous Solution, Plenum Press, New York & London (1996). Typically, the compositions may comprise one or more chelators in a total amount of from 0.0010 to 1.0, in particular from 0.010 to 0.50 percent-by-weight, based on the weight of the The topically applicable rinse-off composition (RC).

The one or more chelators may be selected from the group consisting of carboxylic acids (such as aminocarboxylic acids), phosphonic acids (such as aminophosphonic acids), polyphosphoric acids (such as linear polyphosphoric acids), their salts thereof, and mixtures thereof. By "salts thereof", it is meant - in the context of chelators - all salts comprising the same functional structure as the chelators they are referring to and including alkali metal salts, alkaline earth salts, ammonium salts, substituted ammonium salts, and mixtures thereof; alternatively sodium salts, potassium salts, calcium salts, magnesium salts, ammonium salts, and mixtures thereof; alternatively monoethanolammonium salts, diethanolammonium salts, triethanolammonium salts, and mixtures thereof.

The one or more chelators may be one or more aminocarboxylic acid chelators comprising one or more carboxylic acid moieties (-COOH) and one or more nitrogen atoms. The one or more aminocarboxylic acid chelators may be selected from the group consisting of diethylenetriamine pentaacetic acid (DTPA), ethylenediamine disuccinic acid (EDDS), ethylenediamine-N,N'-diglutaric acid (EDDG), 2-hydroxypropylenediamine-N-N'-disuccinic acid (HPDDS), glycinamide-N,N'-disuccinic acid (GADS), ethylenediamine-N-N'-diglutaric acid (EDDG), 2-hydroxypropylenediamine-N-N'-disuccinic acid (HPDDS), ethylenediaminetetraacetic acid (EDTA), ethylenedicysteic acid (EDC), ethylenediamine-N-N'-bis(ortho-hydroxyphenyl acetic acid) (EDDHA), diaminoalkyldi(sulfosuccinic acids) (DDS), N,N'-bis(2-hydroxybenzyl)ethylenediamine-N,N'-diacetic acid (HBED), their salts thereof, and mixtures thereof. Conveniently, a chelator used in compositions according to the present disclosure, if any, may be EDTA.

The topically applicable rinse-off composition (RC) used in accordance with the present disclosure may comprise viscosity modifiers, referred to in the art as well as "thickening agents". If present, such viscosity modifiers typicall wil be used in the topically applicable rinse-off composition (RC) in an amount of from 0.010 to 3.0, in particular from 0.50 to 1.0 percent-by-weight, based on the weight of the topically applicable rinse-off composition (RC). Suitable cosmetically acceptable viscosity modifiers include non-ionic polymers, for example polysaccharides such as cellulose, cellulose gum or xanthan gum, and non-ionic viscosity modifiers based on polyether-1 such as the commercially available viscosity modifiers Pure Thix 1442.

The topically applicable rinse-off composition (RC) used in accordance with the present disclosure may further comprise care materials, such as hair-care providing or skin-care-providing plant or vegetable extracts, cationic polymers or cationic surfactants. Typical amounts of care materials in the compositions according to the present disclosure will be in the range of from 0.010 to 5.0, in particular 0.050-2.0 percent-by-weight, based on the weight of the the topically applicable rinse-off composition (RC).

Additional cosmetically acceptable additives, which may be present in the topically applicable rinse-off composition (RC) used according to the present disclosure include:
- physiologically compatible silicone derivative compounds, such as volatile or non-volatile silicone or high molecular weight siloxane polymers, such as for example Quaternium-80, typically in an amount of from 0.05 to 20 percent-by-weight, in particular from 0.10 to 5.0 percent-by-weight, based on the weight of the composition
- light protecting agents, such as for example octylmethoxy cinnamate
- anti-flaking agents, typically in an amount of about 0.01 to 2 percent-by-weight
- hair luster-imparting agents, such as for example phenyltrimethicone,
- combability improving agents, such as for example cationic surfactants such as Behentrimonium Chloride, or cationic polymers such as Polyquaternium-10
- defatting agents
- conditioning agents
- moisturizing agents, such as for example sodium lactate, sodium PCA, glycine, fructose, urea, niacinamide, inositol
- cooling agents, such as for example menthol, eucalptol, geraniol, linalool, isopulegol, cubedol, p-mentane-3,8-diols, hydroxy-citronellal as well as esters and derivatives thereof; ketones such as menthone and carvone; carboxamides, or evaporative cooling agents

The cosmetically acceptable additives may be used in the the topically applicable rinse-off composition (RC) used according to the present disclosure in any combinations, as desired or required.

### Substances potentially excluded

The topically applicable rinse-off composition (RC) used according to the present disclosure may be essentially free of, or be free of certain substances or classes of chemical compounds.

According to embodiments, the topically applicable rinse-off composition (RC) used according to the present disclosure may be essentially free of biotin. According to embodiments, the topically applicable rinse-off composition (RC) used according to the present disclosure may be free of biotin. According to particular embodiments, the concentration of biotin may be less than 5 ppm by weight.

According to embodiments, the topically applicable rinse-off composition (RC) used according to the present disclosure may be essentially free of quaternary ammonium compounds. According to embodiments, the topically applicable rinse-off composition (RC) used according to the present disclosure may be free of quaternary ammonium compounds. According to particular embodiments, the concentration of quaternary ammonium compounds may be less than 5 ppm by weight.

According to embodiments, the topically applicable rinse-off composition (RC) used according to the present disclosure may be essentially free of silicones. According to embodiments, compositions according to the present disclosure may be free of silicones. According to particular embodiments, the concentration of silicones may be less than 5 ppm by weight.

According to embodiments, the topically applicable rinse-off composition (RC) used according to the present disclosure may be essentially free of quaternary ammonium compounds and silicones. According to embodiments, the topically applicable rinse-off composition (RC) used according to the present disclosure may be free of quaternary ammonium compounds and silicones. According to particular embodiments, the concentration of quaternary ammonium compounds and silicones may be less than 5 ppm by weight.

According to embodiments, the polymeric compounds present in the topically applicable rinse-off composition (RC) used according to the present disclosure exclusively are film-forming polymers. In other words, according to embodiments, the topically applicable rinse-off composition (RC) used according to the present disclosure may be essentially free of non-film-forming polymers. According to embodiments, the topically applicable rinse-off composition (RC) used according to the present disclosure may be free of non-film-forming polymers. According to particular embodiments, the concentration of non-film-forming polymers may be less than 5 ppm by weight.

According to embodiments, the topically applicable rinse-off composition (RC) used according to the present disclosure may be essentially free of or be free of non-film-forming polymers selected from acrylates/Steareth-20 methacrylate copolymer, carbomer, polyacrylamide, hydroxyethylcellulose, Xanthan Gum and polymers having similar chemistry.

According to embodiments, the topically applicable rinse-off composition (RC) used according to the present disclosure may be essentially free of or be free of unsaturated fatty acids.

### Topically applicable rinse-off composition (RC)

Typically, sandal pentanol (component (A)) will be present in an amount of from 0.010 to 10.0 percent-by-weight, based on the weight of the topically applicable rinse-off composition (RC). Typically, caffeine (component (B)) will be present in an amount of from 0.010 to 10.0 percent-by-weight, based on the weight of the topically applicable rinse-off composition (RC). The alt least one C10-C18 fatty acid and/or salts thereof (component (C)), typically will be present in an amount of from 0.010 to 2.0 percent-by-weight, based on the weight of the the topically applicable rinse-off composition (RC). Conveniently, the sandal pentanol (component (A)) will be present in an amount of from 0.10 to 5.0 percent-by-weight, the caffeine (component (B)) will be present in an amount of from 0.10 to 5.0 percent-by-weight, and the alt least one C10-C18 fatty acid (component (C)) will be present in an amount of from 0.050 to 3.0 percent-by-weight, each based on the weight of the topically applicable rinse-off composition (RC).

According to embodiments, the sandal pentanol (component (A)) will be present in an amount of from 0.50 to 2.0 percent-by-weight, the caffeine (component (B)) will be present in an amount of from 0.50 to 2.0 percent-by-weight, and the at least one C10-C18 fatty acid (component (C)) will be present in an amount of from 0.10 to 2.0 percent-by-weight, each based on the weight of the the topically applicable rinse-off composition (RC). According to more specific embodiments, the sandal pentanol (component (A)) will be present in an amount of from 0.80 to 1.50 percent-by-weight, the caffeine (component (B)) will be present in an amount of from 0.80 to 1.50 percent-by-weight, and the at least one C10-C18 fatty acid (component (C)) will be present in an amount of from 0.10 to 1.0 percent-by-weight, each based on the weight of the topically applicable rinse-off composition (RC).

According to embodiments encompassing the combination of sandal pentanol (component (A)), caffeine (component (B)) and the at least one C10-C18 fatty acid (component (C)), the weight ratio of sandal pentanol to caffeine may be from 1:2 to 2:1, more specifically from 1:1.5 to 1.5:1, and in particular from 1:1.2 to 1.2:1. In such embodiments, the weight ratio of sandal pentanol to the at least one C10-C18 fatty acid may be from 10:1 to 1:1, more specifically from 8:1 to 1.5:1, and in particular from 5:1 to 2:1. Further, the weight ratio of caffeine to the at least one C10-C18 fatty acid analogously may be from 10:1 to 1:1, more specifically from 8:1 to 1.5:1, and in particular from 5:1 to 2:1. Given that the molecular weights of sandal pentanol, caffeine and C10-C18 fatty acids are rather similar, molecular ratios will be similar to the weight ratios.

The topically applicable rinse-off composition (RC) used in the present disclosure can have a pH within the range of from 3.0 to 8.0. According to embodiments, the pH may be within the range of from 4.0 to 6.5, for example within the range of from 4.5 to 6.0.

The topically applicable rinse-off composition (RC) used in the present invention is preferably at least one composition selected from the group consisting of hair lotion, hair conditioner, hair cream, hair shampoo, dry shampoo, hair foam, and shampoo bar.

In a preferred embodiment the topically applicable rinse-off composition (RC) is shampoo or a conditioner.

### Non-therapeutic method

Another aspect of the present invention is the non-therapeutic method for (i) improving hair structure, and (ii) hair loss reduction comprising the following steps:
a) rinsing hair and scalp with water,
b) topically applying the rinse-off composition (RC) as described herein to hair and scalp for a time period of at least 1 minute, preferably at least 2 minutes,
c) rinsing hair and scalp with water to remove the rinse-off composition (RC) as described herein from hair and scalp,
   wherein steps a), b, and c) are conducted at least once in a time period of 48 hours, preferably at least once in a time period of 24 hours.

The term "topically applicable rinse-off composition (RC)" and "composition (RC)" hereinafter will be used synonymously. The topically applicable rinse-off composition (RC) used according to the present disclosure may be employed according to a method wherein the composition (RC) is applied to at least part of the scalp region, or to at least part of a skin region comprising facial hair. Conveniently, the composition (RC) may be applied to at least part of the scalp region, or to at least part of a skin region comprising facial hair and subsequently or simultaneously be gently worked in.

Preferably the composition (RC) is gently worked in after hair and scalp have been rinsed with water. The composition (RC) used according to the present disclosure is applied in an amount sufficient to obtain the desired effects (i and ii). In particular, the composition (RC) may be applied to at least part of the scalp region, or to at least part of a skin region comprising facial hair in order to deliver an effective amount of the active ingredients combination, namely of sandal pentanol (component (A)), caffeine (component B)), and the at least one fatty acid and/or salts thereof (component (C)).

For example, the composition (RC) used according to the present disclosure may be applied to at least part of the scalp region, or to at least part of a skin region comprising facial hair in one, or two, or even three or more applications per day in order to daily deliver a effective amount the active ingredients combination, namely of sandal pentanol (component (A)), caffeine (component B)) and the at least one fatty acid and/or salts thereof (component (C)).

For example, the active ingredients combination, namely of sandal pentanol (component (A)), caffeine (component B)) and the at least one fatty acid and/or salts thereof (component (C)) may be applied to at least part of the scalp region, or to at least part of a skin region comprising facial hair once or twice per day (24 hours).

In case the composition (RC) is a shampoo, it was found to be sufficient applying the composition (RC) once per day (24 hours).

The composition (RC) used according to the present disclosure are preferably topically applied on at least part of the scalp region, or on at least part of a skin region comprising facial hair for at least one minute, preferably for at least 1.5 minutes and more preferably for at least 2 minutes, to obtain the desired effects (i and ii).

In particular, the composition (RC) is preferably removed after use by applying water to hair and scalp.

Further subject matter of the present disclosure is the use of the composition (RC) for improving hair structure (i) and hair loss reduction (ii). For example, the present invention contemplates using the composition (RC) for improving hair structure (i), wherein improving hair structure comprises increasing the cross-section of the hair. Further, the present invention contemplates using the composition (RC), for improving hair structure (i) and hair loss reduction (ii)., wherein improving hair structure comprises increasing the expression of keratin, in particular the expression of keratin 85. Further, the present invention contemplates using the composition (RC) according to the present disclosure, for improving the structure (i) of hair affected by age-related hair thinning.

The following examples should illustrate the invention in greater detail, but the details of these examples should not be considered as limiting the claims.

### Embodiments

1. Cosmetic use of a topically applicable rinse-off composition (RC) comprising as components
   (A) sandal pentanol ((3-methyl-5-(2,2,3-trimethylcyclopent-3-en-1-yl)pentan-2-ol) and /or one or more sandal pentanol analog(s),
   (B) caffeine,
   (C) at least one C10-C18 fatty acid and/or salts thereof,
   (D) at least one surfactant, and
   (E) at least one cosmetically acceptable solvent
   for
   ( i) improving hair structure, and
   ( ii) hair loss reduction,

   wherein the topically applicable rinse-off composition (RC) comprises
   component (A) in an amount of from 0.010 to 10 % by weight,
   component (B) in an amount of from 0.010 to 10 % by weight,
   component (C) in an amount of from 0.010 to 5 % by weight,
   component (D) in an amount of from 5.0 to 30 % by weight, and
   component (E) in an amount of from 45 to 94.97 % by weight,
   wherein the % by weight values are based on the total weight of the topically applicable rinse-off composition (RC).
2. Cosmetic use according to embodiment 1, wherein improving hair structure (i) comprises increasing the cross-section of the hair.
3. Cosmetic use according to embodiments 1 to 2, wherein improving hair structure (i) comprises increasing the expression of keratin.
4. Cosmetic use according to any of embodiments 1 to 3, wherein improving hair structure (i) comprises increasing the expression of keratin 85.
5. Cosmetic use according to any of embodiments 1 to 4, wherein the hair loss reduction (ii) is a reduction of androgenetic alopecia.
6. Cosmetic use according to any of embodiments 1 to 5, wherein the weight ratio of component (A) to component (B) is in the range of from 1:2 to 2:1, more specifically in the range of from 1:1.5 to 1.5:1, and in particular in the range of from 1:1.2 to 1.2:1.
7. Cosmetic use according to any of embodiments 1 to 6, wherein component (D) is at least one detergent surfactant selected from the group consisting of anionic detergent surfactants, cationic detergent surfactants, and non-ionic detergent surfactants, wherein fatty acid surfactants are excluded.
8. Cosmetic use according to any of embodiments 1 to 7, wherein the one or more sandal pentanol analog that may be comprised in component (A) is at least one sandal pentanol analog(s) selected from group consisting of sandal pentenol ((4Z)-3-methyl-5-(2,2,3-trimethylcyclopent-3-en-1-yl)pent-4-en-2-ol), sandal cyclopropane (1-methyl-2-((1,2.2-trimethylbicyclo(3.1.0)hex-3-yl)methyl)-cyclopropane-methanol), santol pentenol ((E)-3,3-dimethyl-5-(2,2,3-trimethylcyclopent-3-en-1-yl)pent-4-en-2-ol), sandal cyclopentane (2-methyl-4-(2,2,3-trimethyl-3-cyclopenten-1-yl)butanol), sandalrome ((E)-2-ethyl-4-(2,2,3-trimethylcyclopent-3-en-1-yl)but-2-en-1-ol), and sandal butenol ((E)-2-methyl-4-(2,2,3-trimethyl-3-cyclopenten-1 -yl)-2-buten-1 -ol).
9. Cosmetic use according to any of embodiments 1 to 8, wherein improving hair structure (i) comprises improving the structure of hair affected by age-related hair thinning.
10. Cosmetic use according to any of embodiments 1 to 9, wherein component (C) comprises at least 50 % by weight of lauric acid and/or salts thereof, based on the total amount of component (C) comprised in the topically applicable rinse-off composition (RC).
11. Cosmetic use according to any of embodiments 1 to 10, wherein component (A) comprises at least 50 % by weight of sandal pentanol, based on the total amount of component (A) comprised in the topically applicable rinse-off composition (RC).
12. Cosmetic use according to any of embodiments 1 to 11, wherein component (E) is at least one cosmetically acceptable solvent selected from the group consisting of water, ethanol, propanol, glycerol, ethylene glycol, and propylene glycol.
13. Cosmetic use according to any of embodiments 1 to 23, wherein the topically applicable rinse-off composition (RC) is at least one composition selected from the group consisting of hair lotion, hair conditioner, hair cream, hair shampoo, dry shampoo, hair foam, and shampoo bar.
14. A non-therapeutic method for (i) improving hair structure, and (ii) hair loss reduction comprising the following steps:
   a) rinsing hair and scalp with water,
   b) topically applying the rinse-off composition (RC) as described in any of embodiments 1 to 13 to hair and scalp for a time period of at least 1 minute, preferably at least 2 minutes,
   c) rinsing hair and scalp with water to remove the rinse-off composition (RC) as described in any of embodiments 1 to 13 from hair and scalp,
      wherein steps a), b, and c) are conducted at least once in a time period of 48 hours, preferably at least once in a time period of 24 hours.

### Examples

### Clinical study results

### Study Methodology

In a monocentric randomized, clinical study the hair status of the test subjects was evaluated by the Trichoscale^{®}: Hair is cut in a small area of about 2 cm² by means of a clipper. The designated area located on lateral parietal region is clipped and the template placed on the area to enable a precise hair shaving. For one subject, the tested area is the same throughout the study. After 3 days, hair is dyed and a video image 20 times enlarged is stored. This image is taken on a 0.903 cm² area (zone of measurement). Trichoscale^{®} measurements provide data on: hair thickness; telogen rate; anagen rate; number of anagen and telogen hair and anagen/telogen ratio.

The anagen rate was measured in the beginning of (T0), and after 12 weeks (T3M), 24 weeks (T6M), 36 weeks (T9M), and after 48 weeks (T12M).

The hair thickness was measured in the beginning of (T0), and after 48 weeks (T12M).

### Study Subjects

For anagen rate the number of included subjects per each leg was 33. All of the included subjects had a telogen rate above 20% at the beginning of the study. The number of subjects finishing the study after 48 weeks were 30 with 50% female and 50% male per each leg from 35 to 65 years old.

For hair thickness the number of included subjects per each leg was 30. All of the included subjects had a telogen rate above 20% at the beginning of the study. The number of subjects finishing the study after 48 weeks were 30 with 50% female and 50% male per each leg from 35 to 65 years old.

### Study Setup

The participants selected for the study used the product by applying the product by massaging it for 2 minutes at home on hair and scalp once a day for 48 weeks.

Hereinafter the formula of the used product is shown:

| | Quantity /% |
|---|---|
| Water Purified (Component (E)) | ad 100 |
| Polyquaternium-10 | 0,1 |
| Citric Acid Anhydrous | 0,32 |

| | |
|---|---|
| Salicylic Acid | 0,2 |
| Methylparaben, NF | 0,1 |
| Sodium Benzoate | 0,48 |
| Trisodium Ethylenediamine Disuccinate | 0,27 |
| Sodium Laureth Sulfate (SLE2S), 70% in water (Component D)) | 15,000 |
| Sodium Lauryl Sulfate (high ph, 29% Active) FFR (Component D)) | 5,00 |
| Cocamidopropyl Betaine High pH Bulk | 6,67 |
| Caffeine Anhydrous (Component (B) | 1 |
| Lauric acid Component (C)) | 0,2 |
| Sandal pentanol (Component (A)) | 1 |
| Mosa Mint Oil | 0,3 |

### Evaluation Criteria

At the above-mentioned time points, the Trichoscale^{®} measurements was performed by an expert. Both anagen rate (% of hair in anagen phase) and hair thickness was analyzed.

The anagen phase is the active growth phase of hair follicles. During the anagen phase, the cells in the root of the hair divide rapidly, adding to the hair shaft. An increase in the anagen rate indicates a hair loss reduction as more hair is in the growth phase.

### Results

The before-after comparison of the hair thickness and anagen rate is statistically significant at all time points compared to the initial ("T0") anagen rate.

Hair thickness does also increase though the measured average after 48 weeks (T12M) of product usage is statistically significant higher versus the initial ("T0") hair thickness:

### Anagen Rate

| **Time** | **n** | **Mean score ± SD** | **Percenta ge variation vs. T0** | **p value*** |
|---|---|---|---|---|
| **T0** | 33 | 69.92 ± 12.22 | - | - |
| **T3M** | 33 | 72.55 ± 12.22 | 2.63 | 0.0036 |
| **T6M** | 33 | 74.96 ± 12.19 | 5.04 | 0.0001 |
| **T9M** | 30 | 79.10 ± 10.93 | 9.11 | <0.0001 |
| **T12M** | 30 | 81.90 ± 11.19 | 11.91 | <0.0001 |

| | | | | |
|---|---|---|---|---|
| *: statistically significant difference versus T0 if p < 0.05 | | | | |

### Hair Thickness / mm

| **Time** | **n** | **Mean score ± SD** | **Percentage variation vs. T0** | **p value*** |
|---|---|---|---|---|
| **T0** | 30 | 0.058 ± 0.01 | - | - |
| **T12M** | 30 | 0.063 ± 0.009 | 8.6 | 0.0003 |

| | | | | |
|---|---|---|---|---|
| *: statistically significant difference versus T0 if p < 0.05 | | | | |

## Claims

1. Cosmetic use of a topically applicable rinse-off composition (RC) comprising as components
(A) sandal pentanol ((3-methyl-5-(2,2,3-trimethylcyclopent-3-en-1-yl)pentan-2-ol) and /or one or more sandal pentanol analog(s),
(B) caffeine,
(C) at least one C10-C18 fatty acid and/or salts thereof,
(D) at least one surfactant, and
(E) at least one cosmetically acceptable solvent
for
(i) improving hair structure, and
(ii) hair loss reduction 1,
wherein the topically applicable rinse-off composition (RC) comprises
component (A) in an amount of from 0.010 to 10 % by weight,
component (B) in an amount of from 0.010 to 10 % by weight,
component (C) in an amount of from 0.010 to 5 % by weight,
component (D) in an amount of from 5.0 to 30 % by weight, and
component (E) in an amount of from 45 to 94.97 % by weight,
wherein the % by weight values are based on the total weight of the topically applicable rinse-off composition (RC).

2. Cosmetic use according to claim 1, wherein improving hair structure (i) comprises increasing the cross-section of the hair.

3. Cosmetic use according to claims 1 or 2, wherein improving hair structure (i) comprises increasing the expression of keratin.

4. Cosmetic use according to any of claims 1 to 3, wherein improving hair structure (i) comprises increasing the expression of keratin 85.

5. Cosmetic use according to any of claims 1 to 4, wherein the hair loss reduction (ii) is a reduction of androgenetic alopecia.

6. Cosmetic use according to any of claims 1 to 5, wherein the weight ratio of component (A) to component (B) is in the range of from 1:2 to 2:1, more specifically in the range of from 1:1.5 to 1.5:1, and in particular in the range of from 1:1.2 to 1.2:1.

7. Cosmetic use according to any of claims 1 to 6, wherein component (D) is at least one detergent surfactant selected from the group consisting of anionic detergent surfactants, cationic detergent surfactants, and non-ionic detergent surfactants, wherein fatty acid surfactants are excluded.

8. Cosmetic use according to any of claims 1 to 7, wherein the one or more sandal pentanol analog that may be comprised in component (A) is at least one sandal pentanol analog(s) selected from group consisting of sandal pentenol ((4Z)-3-methyl-5-(2,2,3-trimethylcyclopent-3-en-1-yl)pent-4-en-2-ol), sandal cyclopropane (1-methyl-2-((1,2.2-trimethylbicyclo(3.1.0)hex-3-yl)methyl)-cyclopropane-methanol), santol pentenol ((E)-3,3-dimethyl-5-(2,2,3-trimethylcyclopent-3-en-1-yl)pent-4-en-2-ol), sandal cyclopentane (2-methyl-4-(2,2,3-trimethyl-3-cyclopenten-1-yl)butanol), sandalrome ((E)-2-ethyl-4-(2,2,3-trimethylcyclopent-3-en-1-yl)but-2-en-1-ol), and sandal butenol ((E)-2-methyl-4-(2,2,3-trimethyl-3-cyclopenten-1 -yl)-2-buten-1 -ol).

9. Cosmetic use according to any of claims 1 to 8, wherein improving hair structure (i) comprises improving the structure of hair affected by age-related hair thinning.

10. Cosmetic use according to any of claims 1 to 9, wherein component (C) comprises at least 50 % by weight of lauric acid and/or salts thereof, based on the total amount of component (C) comprised in the topically applicable rinse-off composition (RC).

11. Cosmetic use according to any of claims 1 to 10, wherein component (A) comprises at least 50 % by weight of sandal pentanol, based on the total amount of component (A) comprised in the topically applicable rinse-off composition (RC).

12. Cosmetic use according to any of claims 1 to 11, wherein component (E) is at least one cosmetically acceptable solvent selected from the group consisting of water, ethanol, propanol, glycerol, ethylene glycol, and propylene glycol.

13. Cosmetic use according to any of claims 1 to 12, wherein the topically applicable rinse-off composition (RC) is at least one composition selected from the group consisting of hair lotion, hair conditioner, hair cream, hair shampoo, dry shampoo, hair foam, and shampoo bar.

14. A non-therapeutic method for (i) improving hair structure, and (ii) hair loss reduction comprising the following steps:
a) rinsing hair and scalp with water,
b) topically applying the rinse-off composition (RC) as described in any of claims 1 to 13 to hair and scalp for a time period of at least 1 minute, preferably at least 2 minutes,
c) rinsing hair and scalp with water to remove the rinse-off composition (RC) as described in any of claims 1 to 13 from hair and scalp,
wherein steps a), b, and c) are conducted at least once in a time period of 48 hours, preferably at least once in a time period of 24 hours.
